# EUROPÄISCHE PATENTSCHRIFT

(11) **EP 2 723 447 B1**
(45) Veröffentlichungstag und Bekanntmachung des Hinweises auf die Patenterteilung: **23.09.2015**
(21) Anmeldenummer: 12740039.8
(22) Anmeldetag: 06.06.2012
(51) Int. Cl.: A61N 1/40, H05H 1/00

(54) **ELEKTRODENANORDNUNG FÜR EINE DIELEKTRISCH BEHINDERTE GASENTLADUNG**
ELECTRODE ARRANGEMENT FOR A DIELECTRICALLY LIMITED GAS DISCHARGE
AGENCEMENT D'ÉLECTRODE POUR UNE DÉCHARGE GAZEUSE À BARRIÈRE DIÉLECTRIQUE

(30) Priorität: 23.06.2011 DE 102011105713
(43) Veröffentlichungstag der Anmeldung: 30.04.2014
(73) Patentinhaber: Cinogy GmbH, 37115 Duderstadt (DE)
(72) Erfinder: BUSSE, Benedikt, 37079 Göttingen (DE); TRUTWIG, Leonhard, 37115 Duderstadt (DE); WANDKE, Dirk, 37308 Heilbad Heiligenstadt (DE); SEGL, Maximilian, 37115 Duderstadt (DE); KOPP, Matthias, 37434 Gieboldehausen (DE); NOLTE, Michael, 37136 Seeburg (DE); SCHARF, Johannes, 51467 Bergisch-Gladbach (DE); STORCK, Karl-Otto, 37115 Duderstadt (DE)
(74) Vertreter: Lins, Edgar
(86) Internationale Anmeldenummer: PCT/DE2012/000602
(87) Internationale Veröffentlichungsnummer: WO 2012/175066

(56) Entgegenhaltungen:
- WO-A1-98/49368
- WO-A2-01/02291
- DE-A1- 10 047 688
- DE-A1-102008 006 256
- DE-U1-202009 011 521

## Beschreibung

Die Erfindung betrifft eine flexible flächige Elektrodenanordnung für eine dielektrisch behinderte Gasentladung, mit einem zentralen Bereich und einem Randbereich und mit einer ein Hochspannungspotential führenden flächigen Elektrode, die in einem flächigen eine Oberseite und eine Anlageseite bildenden Dielektrikum eingebettet ist.

Dielektrisch behinderte Plasmaentladungen werden für zahlreiche Anwendungsfälle eingesetzt. Durch DE 195 32 105 C2 ist es bekannt, die Oberfläche von dreidimensionalen Werkstücken zu behandeln, beispielsweise zu aktivieren oder zu reinigen. Durch eine sogenannte Barriereentladung ist es möglich, eine Ölschicht bis auf minimale Ölbeläge zu reduzieren. Wesentlich ist dabei allerdings, dass eine gleichmäßige Behandlung der Oberfläche stattfindet. Hierfür ist eine homogene Ausbildung des Plasmas erforderlich, wobei die Vorstellung besteht, dass die Plasmaentladungen in voneinander beabstandeten dünnen Filamenten stattfinden. Problematisch ist dies bei unregelmäßig dreidimensional geformten Oberflächen. In der DE 195 32 105 C2 ist daher vorgesehen, von der Oberfläche des Werkstücks eine Negativform mit dem Dielektrikum auszubilden, das somit aus einem formbaren, beispielsweise pressbaren oder tiefziehbaren Kunststoff besteht. Vorgesehen ist dabei ferner, dass eine Zwischenschicht verwendet wird, sodass das Dielektrikum mit der Zwischenschicht unmittelbar an der Oberfläche des Werkstücks geformt werden kann. Die Zwischenschicht wird dann entfernt, um einen Zwischenraum zwischen dem Dielektrikum und der Elektrode zu gewährleisten, in dem sich das Plasma ausbilden kann. Das Dielektrikum wird auf seiner von der zu behandelnden Oberfläche abgewandten Seite mit einem leitenden Material beschichtet, dem die benötigte hohe Spannung in Form einer Wechselspannung zuführbar ist.

Durch DE 10 2007 030 915 A1 ist es bekannt, Hohlfasern aus einem dielektrischen Werkstoff auszubilden, die in ihrem Inneren mit einer metallisch leitenden Beschichtung versehen werden, sodass die Hohlfaser ein Dielektrikum mit einer inneren abgeschirmten Elektrode bildet, das zusammen mit der als Gegenelektrode dienenden Oberfläche eines leitenden Körpers ein Plasmafeld ausbilden kann. Dabei ist es auch vorgesehen, mit den Hohlfasern ein Gewebe auszubilden, das flächig auf eine unregelmäßige Oberfläche, insbesondere Hautoberfläche eines menschlichen Körpers auflegbar ist. Dadurch entsteht der Vorteil einer an die unregelmäßige Topologie der Hautoberfläche anpassbaren Elektrodenanordnung für die Durchführung einer Plasmabehandlung. Nachteilig hieran ist jedoch der hohe Fertigungsaufwand für die Ausbildung der das Gewebe bildenden Hohlfasern, die in ihrem Hohlraum eine flexible Elektrode aufweisen sollen, um die für die Anpassung an die Hautoberfläche erforderliche Flexibilität des Elektrodengewebes zu gewährleisten.

Eine dielektrisch behinderte Gasentladung oder Plasmabehandlung soll erfindungsgemäß auch für unregelmäßig dreidimensional geformte Körper möglich sein, insbesondere auch für die Hautoberfläche eines Lebewesens. Dabei kann die zu behandelnde Oberfläche als Gegenelektrode fungieren, indem sie beispielsweise elektrisch an Masse gelegt ist. Es ist aber auch möglich, mit der Elektrodenanordnung eine eigene Gegenelektrode auszubilden, sodass eine dielektrisch behinderte Plasmabehandlung für unregelmäßig geformte flächige Körper auch in einem Zwischenraum zwischen der Elektrodenanordnung und der Gegenelektrode möglich ist.

Ein produktionstechnisches Problem besteht für die flächigen Elektroden darin, dass die Größe der zu behandelnden Oberfläche oft nicht vorbekannt ist. Es ist daher bekannt, flächige Elektroden in vorbestimmten - ggf. mehreren - Größen herzustellen. Dabei muss ggf. eine Diskrepanz zwischen der Größe der Elektrode und der Größe der zu behandelnden Oberfläche in Kauf genommen werden. Um die komplette Oberfläche zu behandeln muss ggf. die Elektrodenanordnung mehrfach in unterschiedlichen Positionen aufgelegt werden. Ist im umgekehrten Fall die aufgelegte Elektrodenanordnung für die zu behandelnde Oberfläche zu groß, wird ein unnötig großes Feld für die Erzeugung des Plasmas aufgebaut.

Durch WO 01/02291 A2 ist eine flächige Elektrodenanordnung zur Erzeugung einer dielektrisch behinderten Plasmaentladung bekannt, mit der Ozon und/oder Sauerstoffionen in Luft erzeugt werden sollen. Ein flächiger Träger weist dabei auf einer Rückseite eine durchgehende Elektrode in Form einer Beschichtung auf. Auf der Vorderseite ist das Dielektrikum mit einer dünnen dielektrischen Schicht versehen, deren Dielektrizitätskonstante von der des Trägers unterschiedlich ist. Auf der dielektrischen Schicht ist dann eine Gegenelektrode angeordnet, deren flächige Ausdehnung geringer ist als die der dielektrischen Schicht. Die beiden Elektroden werden an eine Hochspannungsquelle angeschlossen. Für die Elektrode auf der Vorderseite ist eine Einspeisung an mehreren hochohmigen Widerständen von der Vorderseite aus beschrieben. In einer Ausführungsform kann der dielektrische Träger aus einem flexiblen Kunststoff, beispielsweise Polyamid bestehen, um in geeigneter Form gefaltet oder aufgerollt werden zu können. Dabei ist auch die Ausbildung einer spiralförmigen Anordnung möglich. Eine Anpassung der Größe der Vorrichtung an einen Anwendungszweck ist weder vorgesehen noch beschrieben.

Der vorliegenden Erfindung liegt die Aufgabe zugrunde, eine flächige flexible Elektrodenanordnung zu schaffen, die einfach und preiswert herzustellen und in ihrer wirksamen Fläche leicht an die Größe der zu behandelnden Oberfläche anpassbar ist.

Diese Aufgabe wird erfindungsgemäß mit einer flexiblen flächigen Elektrodenanordnung der eingangs erwähnten Art dadurch gelöst, dass das flächige Dielektrikum wenigstens im Randbereich die Form eines spiralförmig aufgewickelten flachen Streifens aufweist und die Elektrode durch wenigstens einen in Längsrichtung des aufgewickelten Streifens verlaufenden, in eine Endfläche des Streifens einmündenden elektrischen Leiter gebildet ist, der mit Ausnahme lediglich der Endfläche allseitig von dem Dielektrikum des Streifens begrenzt ist und der im Bereich der Endfläche des Streifens mit einem Kontaktierungselement kontaktiert und gegenüber der Umgebung elektrisch isoliert ist.

Die erfindungsgemäße flächige Elektrodenanordnung lässt sich somit in einer maximal benötigten Größe herstellen und für einen speziellen Anwendungsfall dadurch verkleinern, dass ein Stück des spiralförmig aufgewickelten Streifens abgelängt wird. Die durch den wenigstens einen elektrischen Leiter gebildete Elektrode erstreckt sich in die Endfläche des Streifens und kann sicher und zuverlässig am Ende des Streifens kontaktiert werden, um die Verbindung mit der Hochspannungsquelle zu gewährleisten. Hierfür ist es denkbar, den elektrischen Leiter durch Entfernung des flächigen Dielektrikums zu entisolieren, um dann eine Kontaktierung mit üblichen Verbindungsmitteln herzustellen. Bevorzugt ist jedoch, das Dielektrikum unverändert beizubehalten und die Kontaktierung mit Hilfe von Schneidkontakten herzustellen, die im Endbereich des Streifens von einer großen Oberfläche durch das Dielektrikum hindurchschneiden, um so einen metallischen Kontakt mit dem elektrischen Leiter herzustellen. Eine derartige Kontaktanordnung hat den Vorteil, dass in einfacher Weise eine sichere Isolierung der Elektrodenanordnung bezüglich der dem elektrischen Leiter zugeführten Hochspannung gewährleistet ist.

Der in das flächige Dielektrikum eingebettete elektrische Leiter kann in zahlreichen Ausführungsformen ausgebildet sein. So ist es beispielsweise möglich, den Leiter als einen metallischen Gitterstreifen in den spiralförmig aufgewickelten flachen Dielektrikum-Streifen einzubetten.

In einer produktionstechnisch vorteilhaften Ausführungsform ist der Leiter wenigstens ein schmaler Leiter, der in dem Streifen von der Endfläche zum zentralen Bereich und von dort zur Endfläche zurück verläuft. Ein derartiger schmaler Leiter, insbesondere wenn er als Leiterdraht mit einem runden oder ovalen Querschnitt ausgebildet ist, kann leicht an die Krümmung des spiralförmig aufgewickelten Dielektrikumstreifens angepasst werden und lässt sich darüber hinaus in einfacher Weise wie eine Ader einer Stromleitung kontaktieren. Die erfindungsgemäße Elektrode wird vorzugsweise im Bereich der Endfläche mit einer Kontaktanordnung kontaktiert und gegenüber der Umgebung elektrisch isoliert. Dies gelingt in einfacher Weise mit einer an sich bekannten Kontaktanordnung, die ein einseitig offenes Gehäuse aufweist, in die die Endfläche des spiralförmig aufgewickelten Streifens so eingeschoben ist, dass das Gehäuse die Endfläche, eine Oberseite und Unterseite und schmale Seite eines der Endfläche benachbarten Endbereichs des Streifens isolierend umfasst und die Kontaktanordnung mit in das Dielektrikum von der Oberseite und/oder der Unterseite in das Dielektrikum selbstschneidend eindringenden Schneidkontakte zur Kontaktierung der flächigen Elektrode versehen ist.

Die erfindungsgemäße flexible flächige Elektrodenanordnung ist in einer bevorzugten Ausführungsform zur Anlage auf einer zu behandelnden Oberfläche ausgebildet, wobei die Oberfläche eine Gegenelektrode zu in der Elektrodenanordnung enthaltenen, eine Hochspannung führenden Elektrode bildet. Dabei ist es vorteilhaft, wenn der Streifen auf seiner Anlageseite angeformte, eine Anlagefläche definierende Erhebungen aufweist, deren Zwischenräume als Gasraum zur Ausbildung eines Plasmas geeignet sind. Die Entstehung des Plasmas erfolgt somit in den Zwischenräumen zwischen den Erhebungen des Dielektrikums. In einer weiterhin bevorzugten Ausführungsform sind die Streifen mit das Dielektrikum von der Anlageseite zur Oberseite durchquerenden Durchgangsöffnungen versehen, die vorzugsweise mit einer Absaugeinrichtung für Fluide aus dem durch die Zwischenräume gebildeten Gasraum verbunden werden. Dadurch ist es insbesondere möglich, eine Plasmabehandlung einer Wundoberfläche durchzuführen und beispielsweise Wundsekret über die Durchgangsöffnung während der Plasmabehandlung abzusaugen. Selbstverständlich ist es auch denkbar, über die Durchgangsöffnung geeignete Materialien in den Plasmaraum zwischen den Erhebungen einzuführen, insbesondere gasförmige Materialien.

Auch die erfindungsgemäße Elektrodenanordnung ermöglicht die Ausbildung einer eigenen Gegenelektrode, die Teil der Elektrodenanordnung ist.

Die Erfindung soll im Folgenden anhand von in der Zeichnung dargestellten Ausführungsbeispielen näher erläutert werden. Es zeigen:
- Figur 1: eine schematische perspektivische Ansicht auf eine Anlageseite der Elektrodenanordnung mit einer schematisch dargestellten Verbindung zur einer Hochspannungsversorgung;
- Figur 2: eine schematische perspektivische Ansicht auf eine Oberseite der Elektrodenanordnung gemäß Figur 1;
- Figur 3: eine Draufsicht auf die Anlageseite der Elektrode gemäß Figur 1 mit einer Darstellung der im Inneren der Elektrodenanordnung verlaufenden, eine Elektrode bildenden Leiterdrähte;
- Figur 4: einen Schnitt entlang der Linie A-A aus Figur 3;
- Figur 5: eine schematische perspektivische Darstellung eines weiteren Ausführungsbeispiels einer flächigen Elektrodenanordnung mit einem speziellen Kontaktierungselement zur Kontaktierung der im Bereich einer Endfläche der die Elektrodenanordnung bildenden Streifen durch verlaufenden Leiterdrähte;
- Figur 6: einen Vertikalschnitt entlang der Linie A-A und einer Draufsicht auf die Kontaktierungsanordnung in einem ersten Zustand;
- Figur 7: eine Darstellung der Kontaktierungsanordnung gemäß Figur 6 in einem eingeschobenen Zustand des Streifens;
- Figur 8: eine Darstellung der Kontaktierungsanordnung gemäß Figur 7 in einer heruntergeklappten Stellung eines schwenkbaren Halters für Schneidkontakte;
- Figur 9: eine Darstellung gemäß Figur 8, bei der der schwenkbare Halter durch einen Ihn übergreifenden Schieber verriegelt ist;
- Figur 10: eine schematische perspektivische Darstellung gemäß Figur 5 mit einem kontaktierten und verriegelten Kontaktelement.

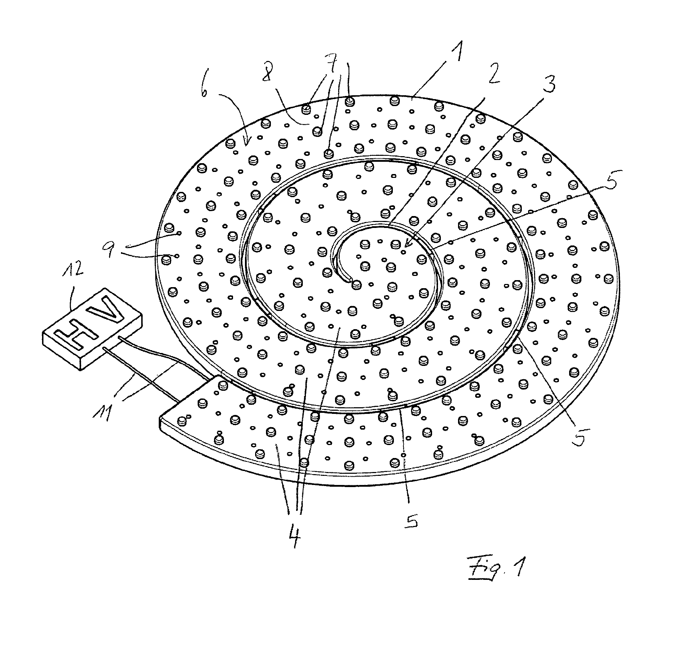

In der in Figur 1 dargestellten ersten Ausführungsform einer erfindungsgemäßen flächigen Elektrodenanordnung besteht diese aus einem spiralförmig ausgewickelten flachen Streifen 1, der mit einem abgerundeten Ende 2 einen zentralen Bereich 3 der Elektrodenanordnung ausbildet und daran anschließend so geformt ist, dass er aneinander anliegende spiralförmige Windungen 4 ausbildet, durch die ein Randbereich definiert wird. Die Windungen des Streifens liegen über angeformte Abstandsstücke 5 aneinander an.

Der Streifen 1 besteht aus einem dielektrischen Material und bildet ein Dielektrikum der Elektrodenanordnung aus. Die in Figur 1 erkennbare Seite stellt eine Anlageseite 6 dar, auf der der Streifen 1 mit einer Vielzahl von Erhebungen 7 versehen ist, die in dem dargestellten Ausführungsbeispiel als runde Noppen ausgebildet sind. Die Erhebungen 7 weisen alle eine gleiche Höhe auf und definieren mit ihrer Oberseite eine Anlagefläche für eine (nicht dargestellte) Gegenelektrode, die durch die zu behandelnde Fläche gebildet sein kann, wenn diese beispielsweise an Masse gelegt ist. Die Erhebungen 7 nehmen nur einen geringen Flächenanteil der Anlagefläche 7 ein, sodass ein weit größerer Anteil der Anlagefläche aus Zwischenräumen 8 zwischen den Erhebungen 7 besteht. Der Flächenanteil der Erhebungen kann zwischen 5 und 40 % der Anlagefläche 6 betragen, während die Zwischenräume 8 einen Flächenanteil von 60 bis 95 %, vorzugsweise zwischen 75 und 95 %, einnehmen.

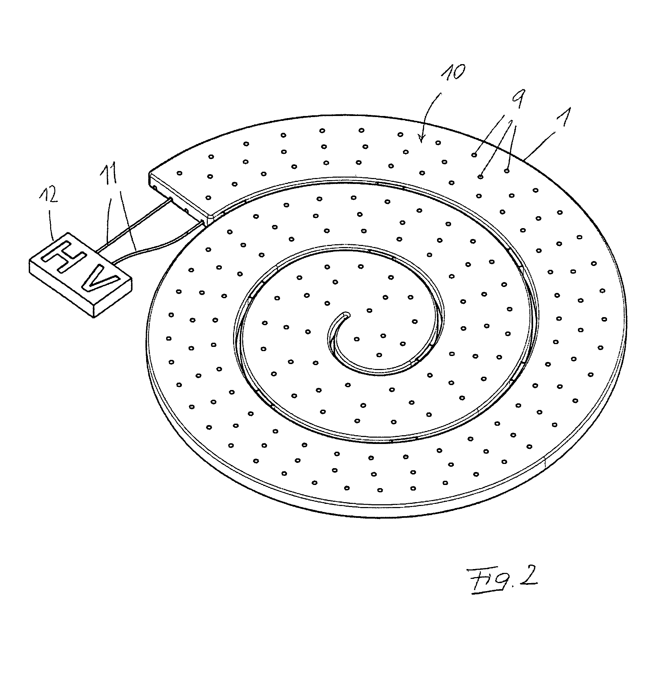

Figur 1 lässt ferner erkennen, dass in dem Streifen 1 kleine Durchgangsöffnungen 9 eingebracht sind, die vorzugsweise gleichmäßig über die Fläche des Streifens verteilt sind. Die Durchgangsöffnungen 9 erstrecken sich von der Anlageseite 6 zur gegenüberliegenden Oberseite 10 (vgl. Figur 2). Die Durchgangsöffnungen 9 erlauben vorteilhafterweise ein Absaugen von Fluid aus den Zwischenräumen 8, in denen sich während des Betriebs der Elektrodenanordnung ein Plasma ausbilden soll.

Zur Ausbildung des Plasmas ist in das durch den Streifen 1 gebildete Dielektrikum eine Elektrode eingebettet, die in Figur 1 mit zwei Leiterdrähten 11 mit einer Hochspannungsversorgung 12 schematisch verbunden dargestellt ist.

Figur 2 zeigt eine Ansicht der Oberseite 10 der durch den Streifen 1 gebildeten Elektrodenanordnung. Von der Oberseite 10 aus sind nur die Durchgangsöffnungen 9 sichtbar.

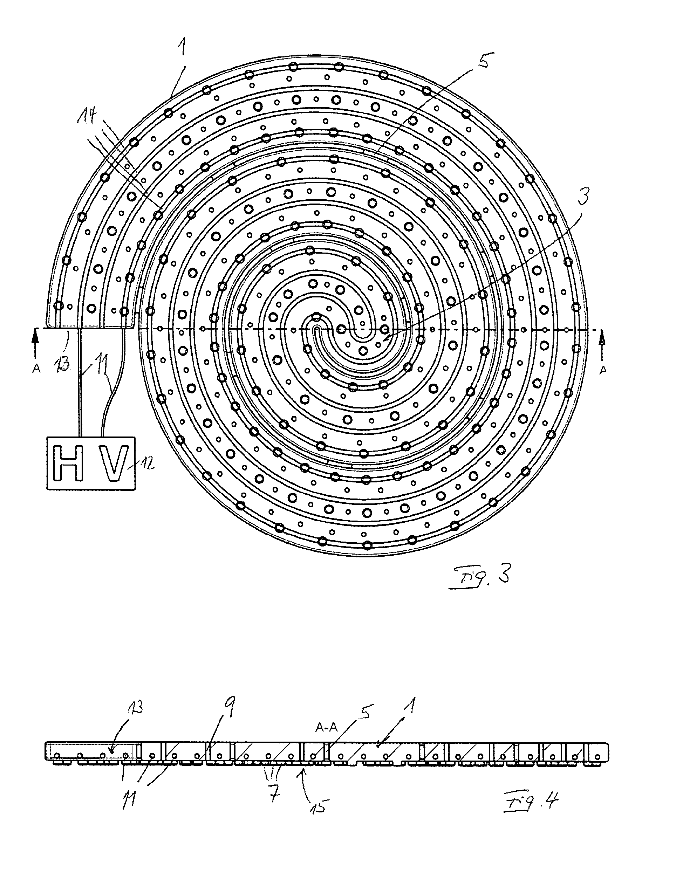

Die Darstellung der Figur 3 lässt erkennen, dass die Leiterdrähte 11 innerhalb des Streifens 1 in dessen Längsrichtung verlaufen, und zwar bis in den durch das abgerundete Ende 2 des Streifens 1 gebildeten zentralen Bereich 3 hinein. In dem zentralen Bereich 3 sind die Leiterdrähte um 180° in Anpassung an das abgerundete Ende 2 umgebogen und verlaufen parallel zu sich selbst durch den Leiter bis zu einer Endfläche 13 zurück. Die Endfläche 13 stellt das äußere freie Ende des Streifens 1 dar und kann rechtwinklig (radial) zu Längserstreckung des Streifens 1 ausgerichtet sein. Es ist aber auch möglich, die Endfläche mit einem beliebigen Winkel zur Längserstreckung des Streifens 1 auszubilden.

Figur 3 lässt erkennen, dass die beiden in Längsrichtung des Streifens 1 hin und her verlaufenden Leiterdrähte 11 eine in das durch den Streifen gebildete Dielektrikum eingebettete Elektrode 14 ausbilden, die somit in dem dargestellten Ausführungsbeispiel durch vier parallel zueinander verlaufende Abschnitte der Leiterdrähte 11 gebildet ist.

Die Leiterdrähte 11 der Elektrode 14 sind mit einem identischen Hochspannungspotential in der Hochspannungsversorgung 12 verbunden. Sie bilden daher eine auf einem Hochspannungspotential liegende Elektrode, die mit einer Gegenelektrode, die beispielsweise durch die zu behandelnde, an Masse liegende Fläche gebildet ist, einen Behandlungsraum, der bei der dargestellten Elektrode durch die Zwischenräume 8 zwischen den Erhebungen 7 gebildet ist. Die Elektrode 14 ist allseitig von dem Dielektrikum der Streifen 1 begrenzt, mit Ausnahme lediglich der Endfläche 13.

Bei der in den Figuren 1 bis 3 schematisch dargestellten Verbindung der Leiterdrähte 11 mit der Hochspannungsversorgung 12 ist selbstverständlich außerhalb der Endfläche 13 eine Isolierung der Leiterdrähte 11 ebenso erforderlich wie an der Endfläche 13 selbst.

Die Schnittdarstellung der Figur 4 lässt eine Ansicht auf die Endfläche 13 erkennen, ferner den durch die Abstandsstücke 5 gebildeten spiralförmig verlaufenden Zwischenraum zwischen den Windungen 4 des Streifens 1 und die Vielzahl der Erhebungen 7, deren gleiche Höhen eine Auflageebene 15 bilden.

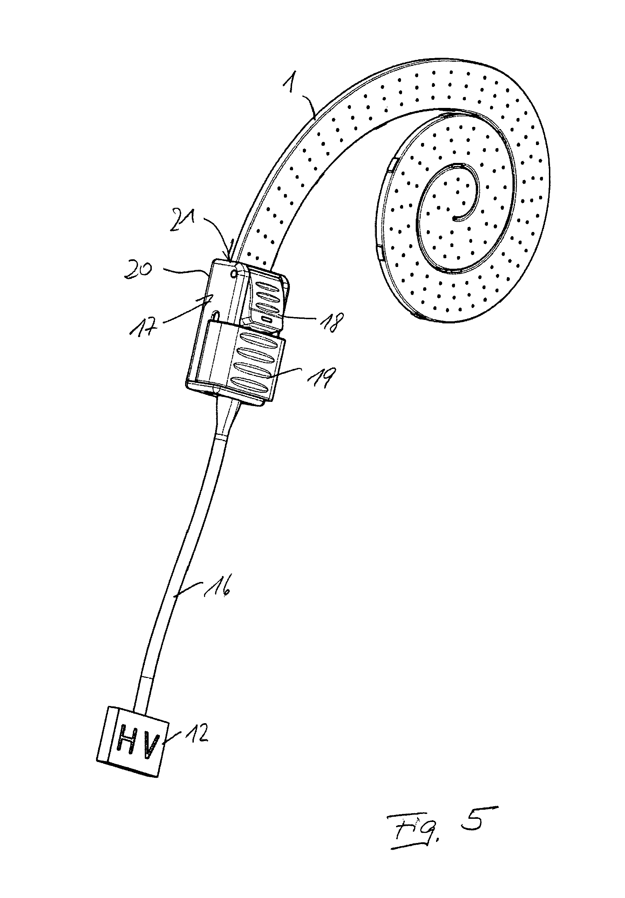

Eine nicht nur schematische, sondern realistische Kontaktierung der innerhalb des Streifens verlaufenden Leiterdrähte 11 ist in Figur 5 dargestellt. Dabei ist die Hochspannungsversorgung 12 über ein isoliertes Hochspannungskabel 16 mit einem Kontaktierungselement 17 verbunden, das einen schwenkbar gelagerten Halter 8 und einen Verriegelungsschieber 19 aufweist. Halter 18 und Verriegelungsschieber 10 befinden sich an einem Gehäuse 20, das allseitig isolierend geschlossen ist und einen Einführungsschlitz 21 für einen die Endfläche 13 aufweisenden Endbereich des Streifens aufweist. Figur 5 verdeutlicht, dass der Endbereich des Streifens in den Einführungsschlitz 21 eingeführt ist.

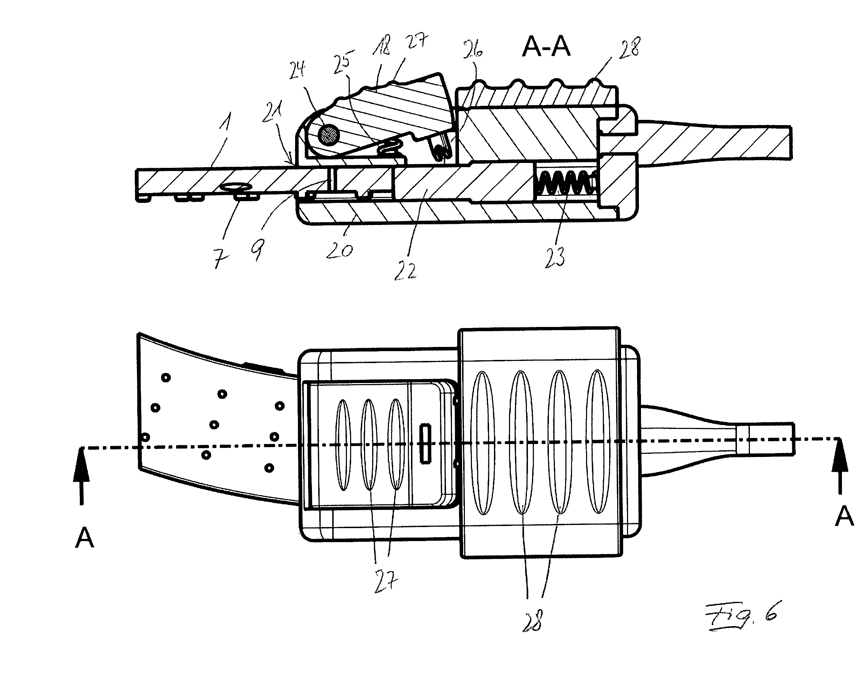

Die Darstellung der Figur 6 zeigt den Endbereich des Streifens 1, der in den Einführungsschlitz 21 des Gehäuses 20 gegen einen Anschlag 22 eingeschoben ist, der längs verschiebbar im Gehäuse 20 gelagert ist und von einer Druckfeder 23 in eine Ruheposition gehalten wird. Die Schnittdarstellung der Figur 6 lässt ferner erkennen, dass der schwenkbare Halter 8 um eine Drehachse 24 verschwenkbar ist, die parallel zum Einführungsschlitz 21, jedoch quer zur Längserstreckung des Streifens ausgerichtet ist. Eine Druckfeder 25 drückt den Halter in eine in Figur 6 dargestellte hochgeschwenkte Position, in der am Ende des Halters 18 an seiner Unterseite gehaltene metallische Schneidkontakte 26 oberhalb des Anschlags 22 liegen.

Figur 6 lässt noch erkennen, dass sowohl am Halter 18 parallele Rippen 27 als auch am Verriegelungsschieber parallele Rippen 28 auf den Oberseiten ausgebildet sind, um die Handhabung zu erleichtern.

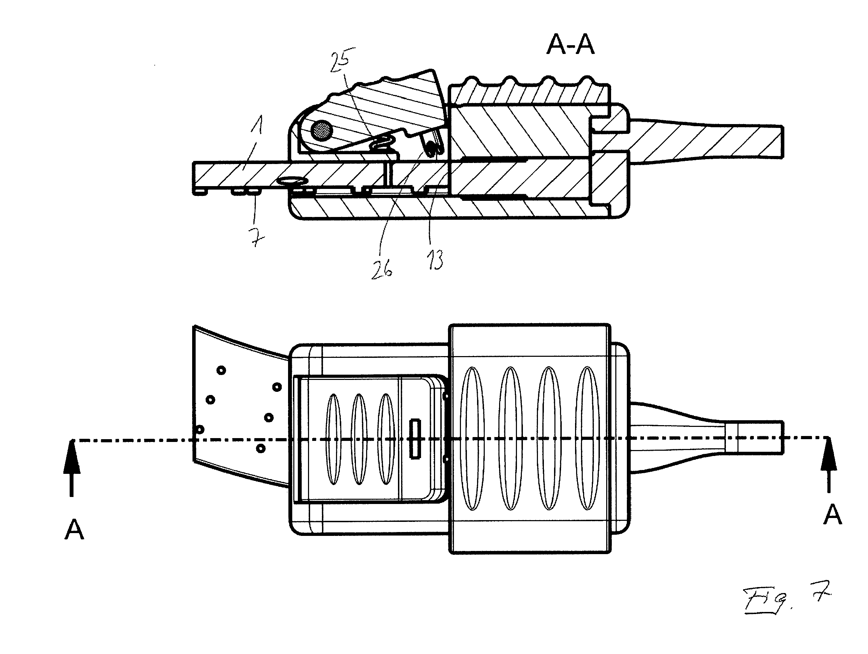

Figur 7 verdeutlicht, dass gegenüber der in Figur 6 dargestellten ersten Position der Streifen 1 weiter in den Einführungsschlitz 21 eingeschoben und dabei den Anschlag 22 gegen die Kraft der in Figur 6 dargestellten Druckfeder 23 axial eindrückt, bis das Ende des Streifens 1 mit der gegen den Anschlag 22 drückenden Endfläche 13 unterhalb der Schneidkontakte 26 zu liegen kommt.

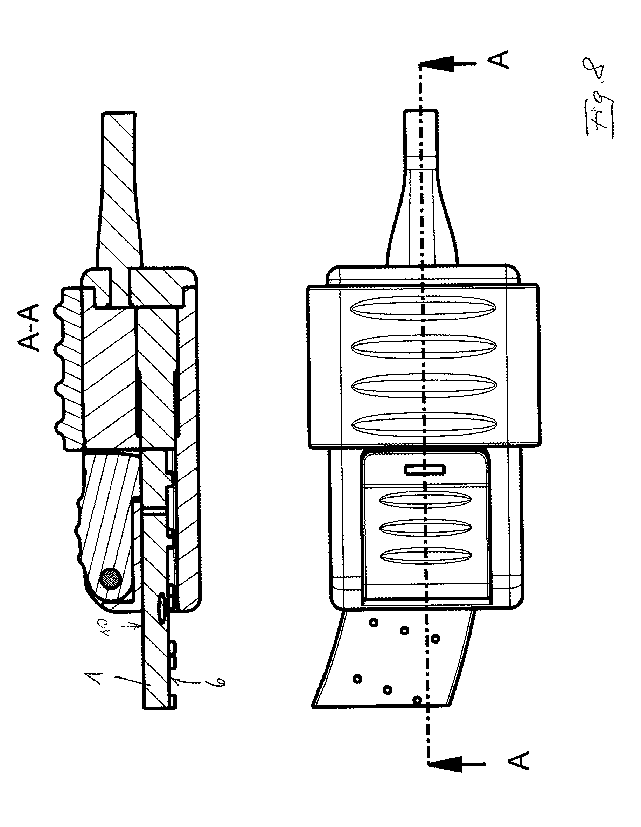

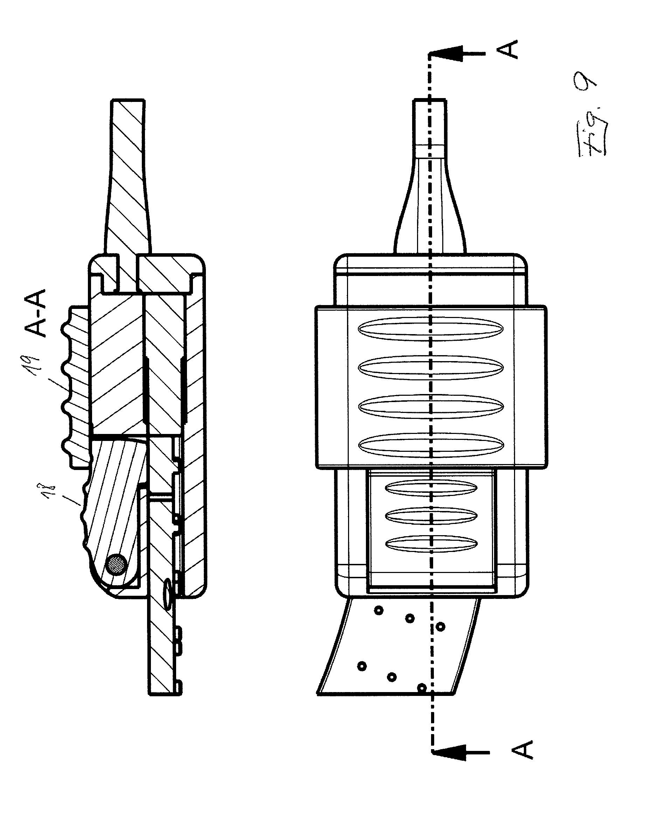

Figur 8 verdeutlicht, dass die Kontaktierung mit den innerhalb des Streifens 1 verlaufenden Leiterdrähten 11 nunmehr dadurch erfolgt, dass der verschwenkbare Halter 18 durch Druck auf seine Oberseite gegen die Kraft der Druckfeder 25 nach unten geschwenkt wird, wodurch die Schneidkontakte 26 von der Oberseite 10 aus in das Material des Streifens 1 einschneidet, bis mit den metallischen Leiterdrähten 11 ein metallischer, d. h. elektrisch leitender, Kontakt hergestellt wird. Unter Beibehaltung des Drucks auf den Halter 18 wird gemäß Figur 9 der Verriegelungsschieber 19 in Längsrichtung verschoben, sodass er nunmehr über den Halter 18 ragt, sodass dieser gegen die Rückstellkraft der Druckfeder 23 in der kontaktierenden Stellung verriegelt ist. Selbstverständlich bestehen Halter 18, Verriegelungsschieber 9, Gehäuse 20 usw. des Kontaktierungselements 17 aus einem nicht leitenden Material, beispielsweise Kunststoff, sodass eine allseitige sichere Isolierung gewährleistet ist und keine Gefahr der nicht behinderten Ableitung der Hochspannung durch ein Berühren von Teilen der Elektrode 14 besteht.

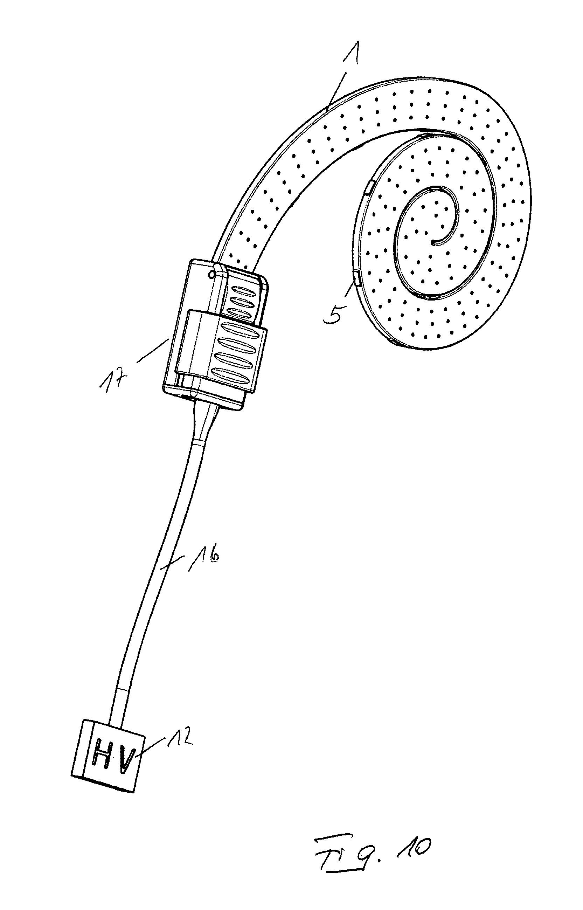

Die vollständig kontaktierte Stellung des Kontaktierungselements 17, das mit der Elektrode 14 des Streifens 1 kontaktiert, ist in Figur 10 dargestellt.

Innerhalb des Kontaktierungselements ist die von der Hochspannungsversorgung 12 über das Hochspannungskabel 16 zugeführte Hochspannungspotential in an sich bekannter Weise zu den Schneidkontakten 26 geleitet, sodass die Elektrode 14 über die Schneidkontakte 26 auf ein Hochspannungspotential gelegt wird.

Es ist ohne weiteres ersichtlich, dass die mit dem Kontaktierungselement 17 durchgeführte Kontaktierung einfach und sicher ist, dass aber andere Kontaktierungsmöglichkeiten, die dem Fachmann geläufig sind, ebenfalls alle Anforderungen an die sichere Isolierung des Hochspannungspotentials gegenüber Ableitungen durch Berührung gewährleisten.

Der besondere Vorteil der dargestellten Elektrodenanordnung besteht darin, dass der Streifen in einfacher Weise an beliebiger Stelle abgelängt werden kann, um eine Anpassung der Größe der Anlageseite 6 an die Größe einer zu behandelnden Fläche zu ermöglichen. Die Ablängung des Streifens 1 ist ohne Beeinträchtigung der Funktion der Elektrode 14 möglich, weil sich die Elektrode in Längsrichtung des Streifens in diesem erstreckt, vorzugsweise bis in den zentralen Bereich 3 hinein. Die Elektrode 14 ist in einfacher Weise an die durch das Ablängen gebildete Endfläche 13 aufweisenden Endbereich des Streifens kontaktierbar, vorzugsweise mit Hilfe eines Kontaktierungselements 17. Die Anpassung der Flächengröße der Elektrodenanordnung an die für die Behandlung einer Fläche benötigte Wirkfläche ist somit in einfacher Weise und ohne Komplikationen lediglich durch Ablängung des Streifens 1 realisierbar.

Der Streifen 1 besteht vorzugsweise aus flexiblem dielektrischem Material, sodass die mit dem Streifen 1 gebildete Elektrodenanordnung an unregelmäßige dreidimensional verlaufende Oberflächen anpassbar ist. Hierzu trägt auch der spiralförmig verlaufende, durch die Abstandsstücke 5 definierte geringe Abstand zwischen den Windungen 4 des Streifens 1 bei.

Es ist erkennbar, dass in den dargestellten Ausführungsbeispielen vorhandenen Durchgangsöffnungen 9 nicht zwingend erforderlich sind, wenn für bestimmte Anwendungsfälle ein Absaugen eines Fluids aus dem Behandlungsraum, der mit den Zwischenräumen 8 ausgebildet ist, nicht benötigt wird. Es ist jedoch bekannt, dass das Absaugen von Fluid beispielsweise für die Behandlung einer Wunde zur Förderung der Wundheilung vorteilhaft sein kann.

Die in den dargestellten Ausführungsbeispielen gezeigte Realisierung der Elektrode mittels wenigstens eines Leiterdrahts 11 ist für die Durchführung der Erfindung nicht zwingend erforderlich. Es ist beispielsweise möglich, die Elektrode durch ein flexibles, in den Streifen eingebettetes Drahtgitter zu realisieren, das in ähnlicher Weise wie die Leiterdrähte 11 kontakierbar ist, beispielsweise mittels Schneidkontakten 26 eines Kontaktierungselements 17.

Für das dielektrische Material des Streifens 1 kommt jedes ausweichend isolierend flexible Material, beispielsweise Kunststoffmaterial in Frage, insbesondere geschäumtes Material, wobei ein geschlossenzelliger Kunststoff- oder Elastomerschaum bevorzugt ist, weil die Gefahr von elektrischen Kurzschlüssen durch in offene Porenstrukturen eindringende Flüssigkeiten dadurch ausgeschlossen wird. Geeignetes Kunststoffmaterial ist beispielsweise geschäumtes Polyurethan oder Silika oder ein geschlossenzelliger Kautschuk, ohne dass eine derartige Aufzählung abschließend gemeint wäre.

## Patentansprüche

1. Flexible flächige Elektrodenanordnung für eine dielektrisch behinderte Gasentladung, mit einem zentralen Bereich (3) und einem Randbereich und mit einer ein Hochspannungspotential führenden flächigen Elektrode (14), die in einem flächigen eine Oberseite (10) und eine Anlageseite (6) bildenden Dielektrikum eingebettet ist, **dadurch gekennzeichnet, dass** das flächige Dielektrikum wenigstens im Randbereich die Form eines spiralförmig aufgewickelten flachen Streifens (1) aufweist und die Elektrode (14) durch wenigstens einen in Längsrichtung des aufgewickelten Streifens (1) verlaufenden, in eine Endfläche (13) des Streifens (1) einmündenden elektrischen Leiter gebildet ist, der mit Ausnahme lediglich der Endfläche (13) allseitig von dem Dielektrikum des Streifens (1) begrenzt ist und der im Bereich der Endfläche (13) des Streifens (1) mit einem Kontaktierungselement (17) kontaktiert und gegenüber der Umgebung elektrisch isoliert ist.

2. Elektrodenanordnung nach Anspruch 1, **dadurch gekennzeichnet, dass** der elektrische Leiter wenigstens ein in dem Streifen (1) von der Endfläche (13) zum zentralen Bereich (3) und von dort zur Endfläche (13) zurück verlaufender schmaler Leiter ist.

3. Elektroden nach Anspruch 1 oder 2, **dadurch gekennzeichnet, dass** der elektrische Leiter ein Leiterdraht (11) mit einem runden oder ovalen Querschnitt ist.

4. Elektrodenanordnung nach einem der Ansprüche 1 bis 3, **dadurch gekennzeichnet, dass** das Kontaktierungselement (17) ein einseitig offenes Gehäuse (20) aufweist, in das die Endfläche (13) des Streifens (1) so eingeschoben ist, dass das Gehäuse (20) die Endfläche (13), die Oberseite (10), Anlageseite (6) und schmale Seiten eines der Endfläche (13) benachbarten Endbereichs des Streifens (1) isolierend umfasst und dass das Kontaktierungselement (17) mit in das Dielektrikum von der Oberseite und/oder der Unterseite selbstschneidend eindringenden Schneidkontakten (26) zur Kontaktierung der flächigen Elektrode (14) versehen ist.

5. Elektrodenanordnung nach einem der Ansprüche 1 bis 4, **dadurch gekennzeichnet, dass** der Streifen (1) auf seiner Anlageseite (6) angeformte, eine Anlageebene (15) definierende Erhebungen (7) aufweist, deren Zwischenräume (8) als Gasraum zur Ausbildung eines Plasmas geeignet sind.

6. Elektrodenanordnung nach einem der Ansprüche 1 bis 5, **dadurch gekennzeichnet, dass** der Streifen (1) das Dielektrikum von der Anlageseite (6) zur Oberseite (10) durchquerende Durchgangsöffnungen (9) zur Absaugung von Fluiden versehen ist.

7. Verfahren zur Anpassung der Flächengröße der Elektrode der Elektrodenanordnung nach einem der Ansprüche 1 bis 6, bei dem eine geeignete Länge des Streifens (1) abgelängt und die so gebildete Endfläche (13) mit dem Kontaktierungselement (17) kontaktiert wird.

## Claims

1. Flexible, flat electrode arrangement for a dielectric gas barrier discharge, comprising a central region (3) and a peripheral region and comprising a flat electrode (14) conducting a high voltage potential, which electrode is embedded in a flat dielectric forming an upper side (10) and a abutting side (6), **characterized in that** the flat dielectric has, at least in the peripheral region, the form of a flat strip (1) wound in helical fashion, and the electrode (14) is formed by at least one electrical conductor which runs in the longitudinal direction of the wound-up strip (1), opens out into an end face (13) of the strip (1), is delimited on all sides, with the exception only of the end face (13), by the dielectric of the strip (1), makes contact with a contact-making element (17) in the region of the end face (13) of the strip (1) and is electrically insulated from the surrounding environment.

2. Electrode arrangement according to Claim 1, **characterized in that** the electrical conductor is at least one narrow conductor running in the strip (1) from the end face (13) to the central region (3) and from there back to the end face (13).

3. Electrode arrangement according to Claim 1 or 2, **characterized in that** the electrical conductor is a conductor wire (11) with a round or oval cross section.

4. Electrode arrangement according to one of Claims 1 to 3, **characterized in that** the contact-making element (17) has a housing (20) which is open on one side and into which the end face (13) of the strip (1) is pushed in such a way that the housing (20) surrounds in insulating fashion the end face (13), the upper side (10), the resting side (6) and the narrow sides of an end region of the strip (1), which end region is adjacent to the end face (13), and **in that** the contact-making element (17) is provided with cutting contacts (26) for making contact with the flat electrode (14) which cutting contacts penetrate into the dielectric in self-cutting fashion from the upper side and/or the lower side.

5. Electrode arrangement according to one of Claims 1 to 4, **characterized in that** the strip (1) has integrally formed elevations (7) on its abutting side (6), which elevations define an abutting plane (15) and have interspaces (8) which are suitable as the gas space for forming a plasma.

6. Electrode arrangement according to one of Claims 1 to 5, **characterized in that** the strip (1) has through-openings (9) for removal by suction of fluids, which through-openings pass through the dielectric from the resting side (6) to the upper side (10).

7. Method for matching the areal size of the electrode of the electrode arrangement according to one of Claims 1 to 6, in which a suitable length of the strip (1) is cut to size, and the end face (13) thus formed is brought into contact with the contact-making element (17).

## Revendications

1. Agencement d'électrode surfacique flexible pour une décharge gazeuse entravée par un diélectrique, comportant une zone centrale (3) et une zone de bordure, et comprenant une électrode surfacique (14) qui laisse passer un potentiel à haute tension et qui est noyée dans un diélectrique surfacique formant une face supérieure (10) et une face d'appui (6), **caractérisé en ce que** le diélectrique surfacique présente au moins dans la zone de bordure la forme d'une bande plane (1) enroulée en forme de spirale, et l'électrode (14) est formée par au moins un conducteur électrique qui s'étend en direction longitudinale de la bande enroulée et qui débouche dans une surface terminale (13) de la bande (1), conducteur qui, à l'exception uniquement de la surface terminale (13), est délimité de tous les côtés par le diélectrique de la bande (1) et qui est en contact avec un élément de mise en contact (17) dans la zone de la surface terminale (13) de la bande (1) et est isolé électriquement par rapport à l'environnement.

2. Agencement d'électrode selon la revendication 1, **caractérisé en ce que** le conducteur électrique est au moins un conducteur étroit qui s'étend dans la bande (1) depuis la surface terminale (13) jusqu'à la zone centrale (3) est depuis celle-ci en retour jusqu'à la surface terminale (13).

3. Agencement d'électrode selon la revendication 1 ou 2, **caractérisé en ce que** le conducteur électrique est un fil conducteur (11) avec une section transversale ronde ou ovale.

4. Agencement d'électrode selon l'une des revendications 1 à 3, **caractérisé en ce que** l'élément de mise en contact (17) comprend un boîtier (20) ouvert sur un côté, dans lequel la surface terminale (13) de la bande (1) est enfilée de telle façon que le boîtier (20) reçoit la surface terminale (13), la face supérieure (10), la face d'appui (6) et les petits côtés d'une zone terminale, voisine de la surface terminale (13), de la bande (1) de manière isolante, et **en ce que** l'élément de mise en contact (17) est doté de contacts coupants (26) qui pénètrent de manière autonome par découpe dans le diélectrique depuis la face supérieure et/ou depuis la face inférieure pour la mise en contact de l'électrode surfacique (14).

5. Agencement d'électrode selon l'une des revendications 1 à 4, **caractérisé en ce que** la bande (1) comporte des bosses (7) conformées sur son côté d'appui (6) et définissants un plan d'appui (15), dont les espaces intermédiaires (8) sont appropriés à titre d'espace gazeux pour la production d'un plasma.

6. Agencement d'électrode selon l'une des revendications 1 à 5, **caractérisé en ce que** la bande (1) est dotée d'ouvertures traversantes (9), qui traversent le diélectrique depuis le côté d'appui (6) jusqu'à la face supérieure (10), pour aspirer des fluides.

7. Procédé pour adapter la taille de surface des électrodes de l'agencement d'électrode selon l'une des revendications 1 à 6, dans lequel on découpe à longueur une longueur appropriée de la bande (1) et on met la surface terminale (13) ainsi formée en contact avec l'élément de mise en contact (17).
